Europäisches Patentamt

European Patent Office

Office européen des brevets

(19)

(11) Publication number: **0 139 091**
**A1**

(12) # EUROPEAN PATENT APPLICATION

(21) Application number: **84107686.2**

(22) Date of filing: **03.07.84**

(51) Int. Cl.⁴: **A 61 M 5/14**
**A 61 M 25/00**

(30) Priority: **18.07.83 US 514714**

(43) Date of publication of application:
**02.05.85 Bulletin 85/18**

(84) Designated Contracting States:
**BE DE FR GB IT**

(71) Applicant: **ABBOTT LABORATORIES**
**14th Street and Sheridan Road**
**North Chicago, Illinois 60064(US)**

(72) Inventor: **Chittenden, Richard Marion**
**385 Getchell Avenue**
**Grayslake Illinois 60030(US)**

(74) Representative: **Modiano, Guido et al,**
**MODIANO, JOSIF, PISANTY & STAUB Modiano &**
**Associati Via Meravigli, 16**
**I-20123 Milan(IT)**

(54) Catheter flashback indicator.

(57) A catheter device which will indicate immediate flashback of blood during venipuncture and in turn indicates positive entry into a blood vessel. The catheter flashback indicator (10) utilizes an over-the-needle catheter (30) with a fluid passage (31) between the catheter (30) and the needle (23) and either an aperture (25) which extends through the needle (23) and communicates between the needle passageway (29) and the fluid passage (31) outside the neddle (23) or an aperture through the catheter in communication with the fluid passage. In a preferred manner, longitudinally extending opaque stripes extend over the fluid passage (31) between the catheter (30) and the needle (23) to give a "thermometer effect" so as to in effect magnify or give a rapid indication of blood flow. Unlike prior art devices, the flashback indicator (10) is located proximal to the piercing point (24) of the needle (23) so as to render an immediate indication of entry into a blood vessel.

Fig.2.

EP 0 139 091 A1

Croydon Printing Company Ltd.

-1-

CATHETER FLASHBACK INDICATOR

Background of the Invention

This invention relates to a flashback patency check device to indicate proper entry into a blood vessel in conjunction with a blood or parenteral administration set. More particularly, this invention relates to a flashback indicator for a catheter unit which can give an immediate indication of entry into a vein during venipuncture.

Flashback indicators currently being utilized are those of the type described in Gewecke, U.S. Patent No. 2,868,200. There are manufacturing problems concerned with this particular unit and accordingly an improvement over this type of flashback unit is described in U.S. Patent No. 4,364,383. Both of these prior art patents illustrate flashback devices wherein the flashback chamber is positioned a substantial distance from the entry point of the piercing needle. This positioning by necessity requires additional time for blood flow from the needle point to the flashback chamber in order to serve as an indicator of proper entry into a blood vessel. Recent prior art devices which have any type of fluid opening adjacent the piercing end or point of a needle or cannula are mainly concerned with one-way slit valves such as illustrated in U.S. Patent No. 4,327,722 or fluid reservoirs such as shown in U.S. Patent No. 4,329,985. In U.S. patents 4,318,402 and 3,840,008, the usual holes are placed in catheter units to permit entry or infusion of fluids.

Nowhere in the prior art is there provided a flashback or patency check device which is positioned immediately adjacent the point of entry of the needle and catheter into the blood vessel so that an immediate flashback of blood can be observed as a positive identification that proper entry has been made into a blood vessel. The prior art is either concerned with

0139091

-2-

flashback chambers located away from the entry point into the blood vessel or with apertured catheters which are not concerned with the observation of blood flashback such as liquid drainage or infusion.

It is an advantage of the present invention to provide a flashback unit which will give an immediate indication of entry of a cannula or injector into a blood vessel. Other advantages are a flashback indicator device for a catheter which is easily manufactured; does not require fabrication of new components or parts; can be easily utilized without new techniques; and a flashback unit which does not substantially add to the cost of I.V. administration.

Summary of the Invention

The foregoing advantages are accomplished and the shortcomings of the prior art are overcome by the present flashback or intravenous injection assembly which includes an injector having a pointed end and defining a wall with a passageway extending inwardly from the pointed end. A transparent or translucent tubular catheter is positioned over the injector and with at least one portion in fluid-tight engagement therewith. The tubular catheter is constructed and arranged to provide a fluid passage means in conjunction with the injector. In one embodiment an aperture communicates with the passageway and extends through the wall from the passageway to the outside of the injector and with the fluid passage means. In an alternative embodiment, instead of an aperture extending through the wall of the injector an aperture is placed through the wall of the catheter so as to communicate with the fluid passage means.

In a preferred manner, a catheter includes longitudinally extending, opaque stripes which extend over the fluid passage means between the catheter and

-3-

the injector so as to give a magnifying or thermometer effect to the presence of blood which indicates proper entry into the blood vessel. The injector can be provided by the usual hollow hypodermic needle or can be a solid-type injector with a passageway extending only a short distance inwardly from the pointed end. The preferred manner of providing a fluid passage means between the catheter and the injector is to utilize a catheter with an inside diameter slightly larger than the outside diameter of the injector. Alternatively a close overfitting catheter could be utilized with a channel in the inside wall thereof to communicate with the aperture through the needle or the catheter. The catheter can be composed of a transparent or translucent material. However, a transparent material is preferred as the presence of blood is more easily observed.

Description of the Drawing

A better understanding of the flashback device of this invention will be accomplished by reference to the drawings wherein:

FIGURE 1 is a view in side elevation of a typical I.V. administration set and solution container with the catheter of the flashback device of this invention attached thereto.

FIGURE 2 is a partial enlarged view of one embodiment of the flashback device of this invention shown in vertical section.

FIGURE 3 is an enlarged partial view in vertical section of the needle with a grinding wheel employed to fabricate an aperture in the needle and illustrating various distances and depths for the grinding wheel and needle by the letters A - E with arrows.

FIGURE 4 is a view in vertical section through the needle taken along line 4-4 of FIGURE 3.

FIGURE 5 is a view similar to FIGURE 2 except showing an alternative embodiment.

FIGURE 6 is a view in vertical section taken along line 6-6 of FIGURE 5.

FIGURE 7 is a view similar to FIGURES 2 and 5 except showing still another embodiment.

FIGURE 8 is a view in side elevation showing a vein in partial view and in vertical section and illustrating the flashback device of this invention in communication therewith.

FIGURE 9 is a view similar to FIGURE 8 except showing a standard prior art unit in communication with a vein for showing a standard flashback procedure.

Description of One Embodiment

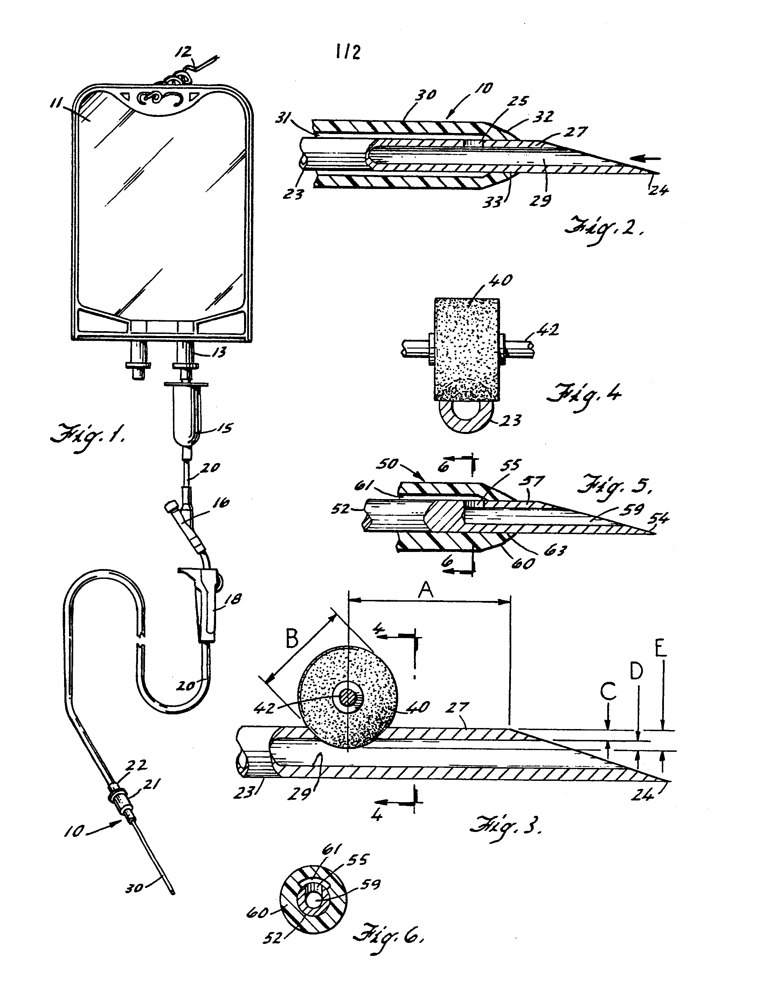

Proceeding to a detailed description of the present invention, the flashback device 10 (with the needle removed) is shown in FIGURE 1 in conjunction with a standard I.V. administration set which is interconnected with a flexible solution container 11 suspended from a support 12. Container 11 includes the usual administration port 13 with a combined piercing pin and drip chamber 15 in fluid communication therewith. Extending between the flashback device 10 and the drip chamber 15 are the standard I.V. set components such as a Y reseal unit 16 and a roller clamp 18 positioned on a length of tubing 20. Male luer adapter 22 affords the usual connection between tubing 20 and catheter hub 21. As best seen in FIGURE 2, the flashback device generally 10 includes a hypodermic needle 23 surrounded by a catheter 30 which in this instance has a slightly larger internal diameter than the external diameter of needle 23 to provide a fluid passage 31. Needle 23 has the usual pointed end 24 and passageway 29. An aperture 25 extends through the wall 27 of the needle to provide communication between passageway 29 and passage 31.

FIGURES 3 and 4 illustrate the preferred means by which aperture 25 can be placed through wall 27 of needle 23. A grinding wheel 40 rotated by shaft 42 is lowered onto the outer surface of the needle and through wall 27 to provide passage 25. A concave aperture will result as viewed from the outside of the needle. In a preferred manner, the diameter of wheel 40 as indicated by distance arrow B will be 0.125 inch and the distance from the end of needle 23 to the center of wheel 40 as illustrated by distance arrow A is 0.200 inch. Assuming that a 27 gauge needle is utilized, the wall thickness as indicated by arrows C will be 0.004 inch, the depth of grind as indicated by arrows E will be 0.006 - 0.007 inch and the distance of penetration into passageway 29 as shown by arrows D will be 0.002 inch. The approximate area of aperture 25 will be 0.000185 square inch.

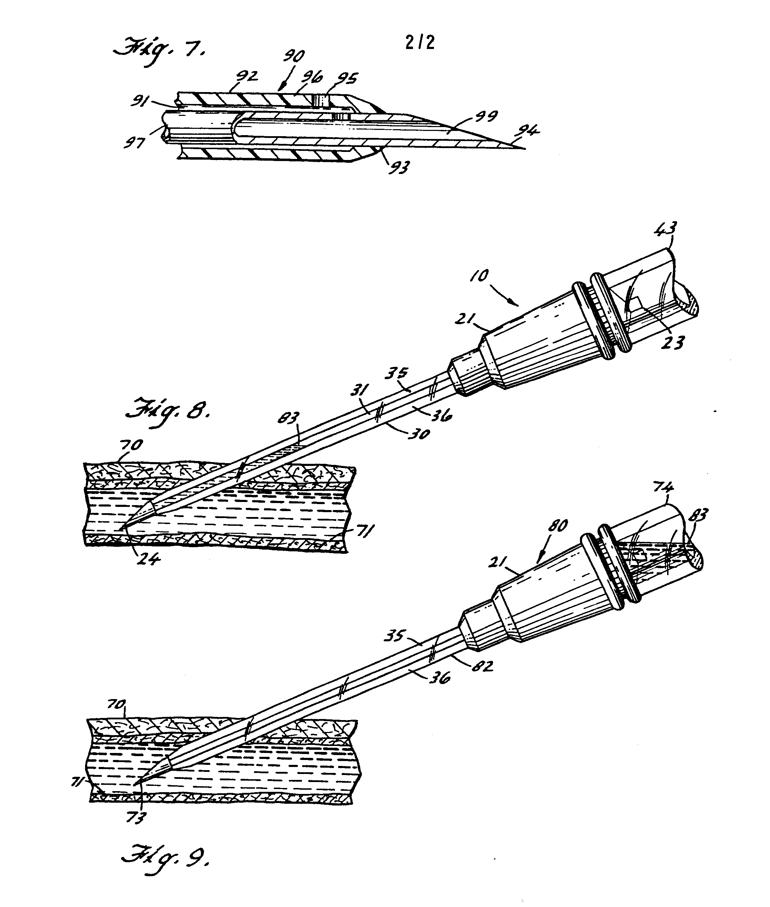

FIGURE 5 illustrates an alternative embodiment of a flashback device 50. Instead of a hypodermic needle such as 23 having a hollow passageway the entire length, passageway 59 extends only a short distance inwardly to communicate with aperture 55 which extends through the needle wall 57. Another distinction between flashback units 50 and 10 is indicated in FIGURE 6. It will be seen that an arcuate-like passageway 61 is illustrated which extends only over a portion of the outside of injector 52. This passage can be provided by a channel molded into the inside wall of catheter 60. In all other respects, catheter 60 will fit snugly over the outside of injector 52.

FIGURE 7 illustrates still a further embodiment 90 of the flashback unit. This particular unit differs from those preceding in that instead of the aperture being placed through the wall of an injector or a hypodermic needle, the aperture 95 is placed through the wall 96 of catheter 92 so as to be in fluid communication with fluid passage 91 formed between a standard hypodermic needle 97 and catheter 92. Placement of catheter 92 over hypodermic needle 97 is similar to the flashback unit 10 in this instance. It will be seen that aperture 95 extends completely through catheter wall 96 and adjacent the point of contact 93 of catheter 92 over needle 97 having the usual pointed end 94.

Operation

A better understanding of the advantages of flashback indicators 10, 50 and 90 will be had by a description of their application and operation, particularly in conjunction with FIGURES 8 and 9. FIGURE 8 illustrates the use of the indicator of this invention and FIGURE 9 shows the standard method of observing flashback. As explained previously, aperture 25 can be formed in a standard hypodermic needle 23 by means of grinding wheel 40 as illustrated in FIGURE 3. The same procedure can be used to supply aperture 55 in needle injector 52. With respect to flashback unit 10, once aperture 25 is placed through the needle wall 27 a catheter 30 will be selected so that it has a diameter slightly larger than the outside diameter of needle 23. When a 27 gauge needle is utilized with an outside diameter of .016 inch, a catheter with an inside area of 0.0000636 inch has been found to work well to provide fluid passage 31. Catheter 30 will be positioned so that the point or tip 32 is adjacent aperture 25. As seen in FIGURE 8, catheter 30 will be connected to the usual catheter adapter 21 at the end opposite point 24 and internal needle 23 will have the standard hub 43. The flashback device can be packaged in the usual manner in a sterile container. When ready for use, the usual venipuncture will be made with point 24 entering the lumen 71 of blood vessel 70 as illustrated in FIGURE 8. When proper entry is made, blood will flow past point 24 into needle passageway 29, through aperture 25 and along passage 31. As catheter 30 is made of a transparent and translucent material, the blood is immediately observed indicating a proper venipuncture.

Catheter 30 has two parallel and radiopaque stripes 35 and 36 which extend longitudinally over catheter 30 and consequently over fluid passage 31. These stripes will help to highlight blood flow with the blood being indicated by the numeral 33. This is the so-called "thermometer effect." These opaque stripes of course can be utilized with catheters 60 and 92. After proper entry into a vein is made of the catheter such as 30, needle 23 with hub 43 is removed as is standard practice and the I.V. set male luer adapter 22 will then be inserted into catheter hub 21.

The operation of flashback device 50 is substantially the same as that previously described for flashback unit 10 except that blood will not flow the length of needle or injector 52. Instead it will flow only as far as aperture 55 and into passage 61 where it will be observed. While injector 52 has been described in conjunction with aperture 55, it is obvious that injector 52 could be utilized with catheter 92 and aperture 95 except that it may or may not have aperture 55.

The operation of flashback unit 90 is also similarly employed as with the previous flashback units except in this instance with hole in the catheter 92 the blood flow will be over the outside of catheter 92 through aperture 95 and into passageway 91 where it will be observed. Blood, of course, will also flow through passageway 99 of needle 97. However, it will not be observed because of the needle being composed of the usual stainless steel.

It will be readily apparent that by being able to observe blood flowage in passages such as 31 which are adjacent the point of entry into the blood vessel such as needle point 24 that an immediate indication of the blood flow can be observed. This is in contrast to the use of the usual catheter unit 80 with catheter 82 shown in FIGURE 9 where one must wait until the blood flows into the needle hub 74 to indicate proper entry into vein 70. The importance of this immediate flashback is realized when consideration is given to the fact that in venipunctures today smaller and smaller size catheters for neonates, infants and geriatrics are being employed. These small catheters have even smaller needles with inherent longer flahsback times. By means of the present invention one must not wait until the blood reaches a needle hub or a flashback chamber positioned beyond the hub but instead can visualize it immediately as the needle point enters the vein.

In the previous description it was indicated that catheters 30, 60 and 92 can be composed of a transparent or translucent material. Preferably, this is Teflon. However, other resinous plastics such as polyurethane, polypropylene, or polyethylene could be employed. Radiopaque stripes such as 35 and 36 are commonly utilized in catheters of this particular type. The hypodermic needles such as 23, 97 or injector 52 are composed of stainless steel. While the apertures 25 and 55 were fabricated in needle 23 and injector 52 by means of a grinding operation, they could also be placed therein either by conventional drilling or by laser. Similarly aperture 95 in catheter 92 can be conveniently made therein either by conventional drilling or with a laser. Two opaque stripes 35 and 36 have been indicated for use with catheter 36. If desired, any number of such stripes could be employed including only a single stripe, whether with catheter 30 or 60 and 92. Further, contact points 33, 63 and 93 are illustrated between the catheter and the needles or injector. These are not essential and could be eliminated, although a more efficient unit is presented with them.

It will thus been seen that through the present invention there is now provided a flashback indicator which will provide a fast and reliable indication of entry of a catheter into a blood vessel. The flashback indicator of this invention can be easily fabricated without extensive tooling while utilizing standard component devices. Accordingly, the flashback indicator herein can be provided at a minimum of cost. Further, no new venipuncture technique must be employed in utilizing the flashback indicator.

The foregoing invention can now be practiced by those skilled in the art. Such skilled persons will know that the invention is not necessarily restricted to the particular embodiments presented herein. The scope of the invention is to be defined by the terms of the following claims as given meaning by the preceding description.

CLAIMS:

1. An intravenous injection assembly for indicating entry into a blood vessel and rapid flashback of blood comprising:

an injector having a pointed end and defining a wall with a passageway extending inwardly from said pointed end;

an aperture communicating with said passageway and extending through said wall of said injector in said passageway to the outside of said injector; and

a transparent or translucent tubular catheter positioned over said injector, said tubular catheter constructed and arranged to provide fluid passage means in communication with said aperture;

whereby when said injector with said catheter is injected into a blood vessel, the blood will flow into injector passageway, through said aperture and into said fluid passage means to indicate an immediate entry of said injector into said blood vessel.

2. The intravenous injection assembly as defined in Claim 1 wherein said catheter is constructed to contact said injector between said aperture and said pointed end.

3. The intravenous injection assembly as defined in Claim 1 wherein said injector is defined by a hollow needle.

4. The intravenous injection assembly as defined in Claim 1 wherein said injector is defined by a solid pointed member except for said passageway and said aperture.

5.  The intravenous injection assembly as defined in Claim 1 wherein said fluid passage means is defined by said tubular catheter having an inside diameter slightly larger than the outside diameter of said injector, said catheter including a tip portion in fluid-tight contact with said injector between said pointed end and said aperture.

6.  A method of manufacturing an intravenous injection assembly for indicating blood flashback comprising:

providing an injector having a pointed end and a wall with a passageway extending inwardly from the pointed end;

locating an aperture through said injector wall and in communication with said passageway inwardly from said pointed end; and

positioning a transparent or translucent tubular catheter over said injector and in fluid-tight engagement therewith, with said tubular catheter constructed and arranged to provide fluid passage means in communication with said aperture.

7. An intravenous injection assembly for indicating entry into a blood vessel and rapid flash of blood comprising:

an injector having a pointed end and defining a wall with a passageway extending inwardly from said pointed end;

a transparent or translucent tubular catheter defining a wall positioned over said injector, said tubular catheter constructed and arranged to provide fluid passage means between said catheter and said injector; and

an aperture extending through said catheter wall and communicating with said fluid passage means;

whereby when said injector with said catheter is injected into a blood vessel, the blood will flow through said aperture and into said fluid passage means to indicate an immediate entry of said injector into said blood vessel.

8. The intravenous injection assembly as defined in Claim 7 wherein said catheter is constructed to contact said injector between said aperture and said pointed end.

9. The intravenous injection assembly as defined in Claim 7 wherein said injector is defined by a hollow needle.

10. The intravenous injection assembly as defined in Claim 7 wherein said fluid passage means is defined by said tubular catheter having an inside diameter slightly larger than the outside diameter of said injector, said catheter including a tip portion in fluid-tight contact with said injector between said pointed end and said aperture.

0139091

1/2

Fig. 1.

Fig. 2.

Fig. 3.

Fig. 4

Fig. 5.

Fig. 6.

Fig. 7.

2/2

Fig. 8.

Fig. 9.

European Patent
Office

**EUROPEAN SEARCH REPORT**

Application number

EP 84107686.2

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int. Cl.4) |
| X <br> Y | US - A - 4 317 445 (T.P.ROBINSON) <br> * Totality * <br><br> -- | 7-10 <br> 1-3,5, 6 | A 61 M 5/14 <br> A 61 M 25/00 |
| Y | US - A - 3 500 828 (F.W.PODHORA) <br> * Totality * <br><br> -- | 1-3,5, 6 | |
| A | FR - A1 - 2 308 346 (K.STORZ) <br> * Totality * <br><br> -- | 7-10 | |
| A | US - A - 3 994 287 (G.TURP et al.) <br> * Fig.1-3; column 2, line 40 - column 3, line 57 * <br><br> ---- | 4 | |
| | | | **TECHNICAL FIELDS SEARCHED (Int. Cl.4)** <br><br> A 61 M 5/00 <br> A 61 M 25/00 <br> A 61 B 17/00 |

The present search report has been drawn up for all claims

| Place of search | Date of completion of the search | Examiner |
|---|---|---|
| VIENNA | 10-12-1984 | LUDWIG |